# EUROPEAN PATENT SPECIFICATION

(11) **EP 0 415 321 B1**
(45) Date of publication and mention of the grant of the patent: **08.11.1995**
(21) Application number: 90116388.1
(22) Date of filing: 27.08.1990
(51) Int. Cl.: C07K 2/00, A61K 38/02, A61K 35/60

(54) **Oligodendrocyte cytotoxic factor**
Für Oligodendrocyten cytotoxischer Faktor
Facteur cytotoxique pour les oligodendrocytes

(30) Priority: 28.08.1989 IL 91459
(43) Date of publication of application: 06.03.1991
(73) Proprietor: YEDA RESEARCH AND DEVELOPMENT COMPANY LIMITED, Rehovot 76100 (IL)
(72) Inventor: Schwartz, Michal, Rehovot (IL); Cohen, Avi, Rehovot (IL)
(74) Representative: VOSSIUS & PARTNER

(56) References cited:
- EP-A- 0 172 987
- EP-A- 0 353 780
- BIOLOGICAL ABSTRACTS, vol. 84, no. 4, 1987, abstract no. 38425, Philadelphia,PA, US; N.B. ZAK et al.: "Laminia-immunore-active sites are induced by growth-accociated triggering factors in injured rabbit optic nerve",& BRAIN RES. 408(1/2): 263-266. 1987
- Neuron 1 (1988) 85-96

## Description

The present invention relates to an oligodendrocyte inhibitory/cytotoxic factor capable of causing a reduction in the number of process-bearing oligodendrocytes. The new factor is useful for enhancing regeneration of injured nerves of the central nervous system in mammals.

In the mammalian central nervous system (CNS), neurons do not regenerate severed axons spontaneously, while fish and amphibian neurons readily regenerate their axons. The inability of the mammalian CNS to regenerate does not seem to result from an intrinsic property of their CNS neurons, as these neurons are able to accomplish considerable axonal regrowth in vivo into a peripheral nerve bridge. In in vitro preparations, injured mammalian CNS axons do not grow on or into a segment of mammalian optic nerve, but will grow on or into a fish optic nerve or into a mammalian peripheral nerve. The differences are thus likely to reside in the different environment; mammalian CNS environment does not support growth, while the environment of peripheral nerves or of fish optic nerves does support growth.

The optic nerve is a favorable model for studying the role of glial cells in axonal regeneration, as neuronal cell bodies are absent and the nerve is accessible for lesion. Among the cellular components of the rat optic nerve are the macroglia (astrocytes and oligodendrocytes and microglia (amoeboid and ramified microglia). The astrocytes are divided into two types, which can be distinguished by antigenic markers: the protoplasmic or type-1 astrocytes are Ran2+, GFAP+, and A₂B₅-, while the fibrous or type-2 astrocytes are Ran2-, GFAP+, and A₂B₅+ (Miller and Raff, 1984; Raff and Miller, 1984)). The type-2 astrocytes and oligodendrocytes have a common progenitor cell (the O-2A progenitor), which has the phenotype Ran2-, GFAP-, and A₂B₅+. The oligodendrocyte/type-2 astrocyte lineage (arising from the O-2A progenitor) is specialized for myelination of axons: the oligodendrocytes produce the myelin and the type-2 astrocytes contribute to the structure of the nodes of Ranvier. The O-2A progenitor cells can be made to differentiate in vitro into either oligodendrocytes or type-2 astrocytes depending on the culture medium. The type-1 astrocytes have a different progenitor cell.

The nonneuronal cells contribute to the CNS environment and they have been implicated in the failure of CNS regeneration in mammals. These cells include astrocytes, which hypertrophy to form fibrous scars in response to lesion and oligodendroglia, which are inhibitory to axonal growth. The astrocytic scar is largely composed of type-1 astrocytes and has been considered to prevent growth by forming a physical barrier. The time course of scar formation is long, however, and it seems unlikely that a barrier formed by the scar would prevent regenerative axonal growth in the immediate posttraumatic period. Type-1 astrocytes in rat optic nerve have been shown to express laminin, a molecule implicated in support of axonal outgrowth, prenatally, during the development of optic nerve. Mammalian adult brain type-1 astrocytes normally do not express laminin, except only transiently following an injury to the brain. In contrast, laminin is continuously expressed in regenerative fish optic nerve. In in vitro preparations, axons grow in close contact with type-1 astrocytes.

Mature oligodendrocytes are now believed to be nonpermissive for axonal growth. Growing axons will avoid contacting mature oligodendrocytes, in vitro. During development, most axonal growth in the optic nerve takes place before birth, before any oligodendrocytes have differentiated. It seems, therefore that axonal regeneration in mammals is hindered by both the presence of mature oligodendrocytes, which are nonpermissive to axonal growth, and the type-1 reactive astrocytes, which are lacking the supportive element(s), in contrast to the fish optic nerve.

Prior work of the present inventors and others has shown that the CNS of lower vertebrates, specifically regenerating fish optic nerve, is a source of factors which, when applied at the appropriate time and in appropriate amounts to injured mammalian adult optic nerves, can support regenerative axonal growth. (Schwartz et al., 1985; Hadani et al., 1984; Lavie et al., 1987 and Cohen et al., 1989).

Robbins et al., 1987, have reported that stimulation of rat astrocytes in vitro resulted in the generation of a cytotoxic factor that is functionally similar to tumor necrosis factor. They also report that human recombinant tumor necrosis factor has cytotoxic activity directed against rat oligodendrocytes. Selmaj et al., 1988, reported on the testing of recombinant human tumor necrosis factor (rhTNF) for its effect on myelinated cultures of mouse spinal cord tissue. They found that rhTNF induced delayed-onset oligodendrocyte necrosis and a type of myelin dilatation.

Despite substantial research efforts worldwide, no safe and effective means for causing CNS regeneration in mammals, and particularly humans, has yet been developed. Such a means, and particularly a pharmaceutical which can be injected at the site of desired regeneration would be very desirable in order to alleviate post-traumatic paraplegia or quadraplegia, blindness, deafness, surgically associated axotomy, etc.

The invention relates to an oligodendrocyte cytotoxic factor, herein designated OCF, from lower non-mammalian vertebrates having the following characteristics:
i. it is selectively toxic to oligodendrocytes, but not to other cells, such as type-1 astrocytes and fibroblast cells;
ii. it reduces the number of mature oligodendrocytes in cultures of injured nerves of lower vertebrates and of mammals;
iii. it inhibits differentiation of progenitors of the oligodendrocyte lineage to mature oligodendrocytes;
iv. it is water-soluble;
v. it is heat sensitive, losing its activity on heating at 56°C;
vi. it is protease sensitive losing its activity upon digestion with trypsin; and
vii. it is present in, and is purifiable from, the conditioned media of regenerating injured nerves of lower non-mammalian vertebrates, such as fish, but is not present in the conditioned media of intact nerves of lower vertebrates nor in the conditioned media of injured or intact nerves of mammals.

The invention also relates to a method of isolation of OCF from different sources, such as from regenerating fish optic nerves, and to its purification.

The invention also relates to the use of OCF for the preparation of pharmaceutical compositions useful for regeneration of nerves of the CNS in mammals, and to the pharmaceutical compositions thus obtained.
The figures show:

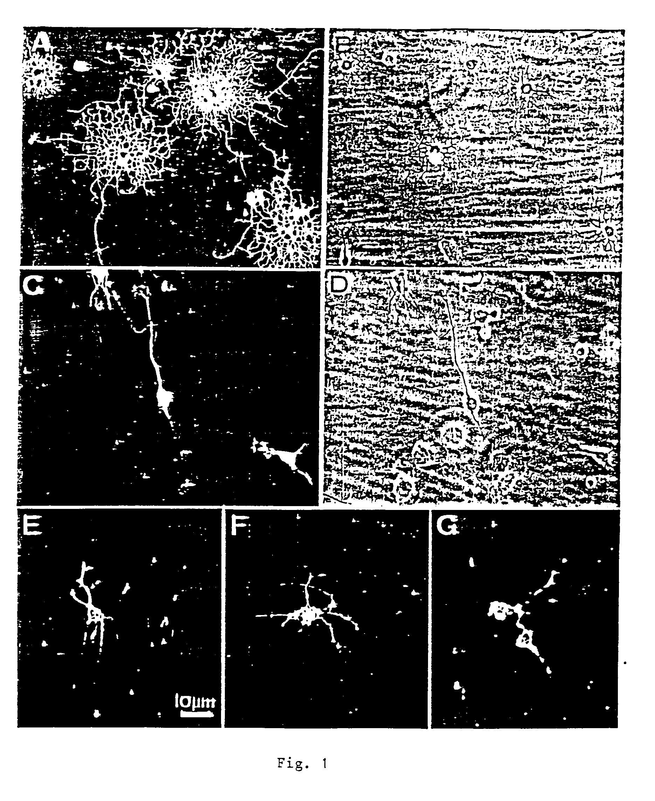

**Figure 1** shows the effect of fish CM-R on adult 04 positive cells in cultures of injured adult rat optic nerves. Cells were prepared from adult rat optic nerves that were crushed 3 days before excision and were seeded on poly-L-lysine coated coverslips in defined medium. Cultures were stained for 04 immunoreactivity at 96 h in vitro, by an indirect immunofluorescent method, which was carried out as follows: Cells were first incubated with mouse anti-04 antibodies for 30 min, followed by 30 min incubation with fluorescein conjugated goat anti-mouse IgM. At the end of the second incubation, the cells were washed and fixed with cold methanol (-20°C) for 10 min. Panels (a) and (b) show, respectively, fluorescent and phase micrographs of the control cells in defined medium without any treatment. Panels (c), (e), (f) and (g) show 04 positive cells in cultures which were treated with CM-R (12 µg protein/ml) for 48 h prior to the staining. Panels (c) and (d) represent fluorescent and phase micrographs of the same cells. Pictures were taken from one experiment, the results of which were reproduced in two additional experiments.
**Figure 2** shows the effect of CM-R on in vitro development of Galc positive cells from 1 day postnatal rat brain. Brains from newborn rats were dissected out and dissociated according to the procedure of McCarthy and DeVellis. After 8 days in vitro, the oligodendrocytes were shaken off and seeded on poly-L-lysine coated coverslips (10⁴ cells/well in a microtiter plate). Cultures were stained at 24, 48 and 72 h in vitro, for Galc immunoreactivity by the direct immunofluorescent technique using fluorescein conjugated goat anti-mouse IgM. In experimental cultures, CM-R (1.2 and 12 µg protein/ml) was added at the indicated time after seeding (marked by an arrow). In each case, the concrete number of Galc positive cells on the entire coverslips was counted (numbers above the everyday graph). In the control culture, the number of Galc positive cells remained relatively constant throughout the experiment (24-72 h) and was about 700-850 cells. Note the lack of inhibitory effect on development of mature oligodendrocytes, at all periods in culture, when CM-R at a concentration of 1.2 µg protein/ml was added to the culture medium at the time of seeding. A marked (more than 50%) inhibitory effect was observed when CM-R at 12 µg protein/ml was added at the time of seeding. When CM-R was added at 24 h in vitro after seeding there was almost 50% inhibition of oligodendrocyte maturation after additional 24 h in vitro, however, this effect seemed to be transient. This experiment was repeated twice and gave qualitatively the same results (N.D.-not done). Fig. 2 shows also that the inhibitory effect was also reproducible when the number of Galc positive cells was monitored by ELISA, rather than by immunofluorescence. Also shown in Fig. 2, is a dose dependency curve of the inhibitory activity as a function of the amount of applied CM-R.

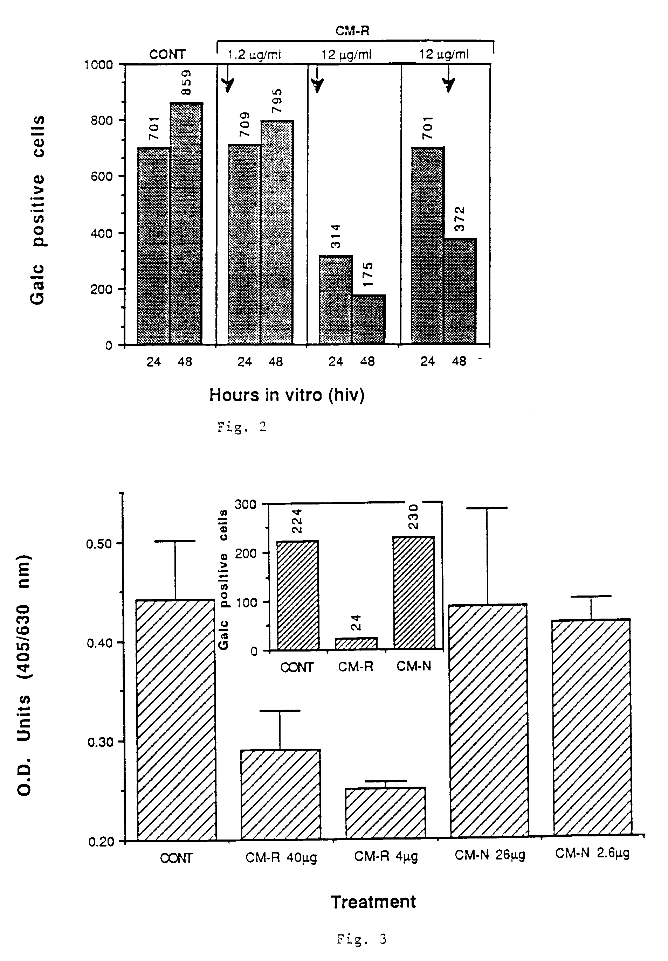

**Figure 3** shows a comparison between the effect of CM-R and CM-N on development of Galc positive cells in cultures of neonatal rat brain oligodendrocytes. Cultures of oligodendrocytes were prepared as above, and seeded in multiwell (10³ cells/microtiter plate). After 24 h in vitro, CM-R or CM-N were added at the indicated concentrations. Forty-eight h later, the cells were examined by incubating first with Galc antibodies at 37°C for 30 min, followed by horseradish peroxidase conjugated to goat anti-mouse antibodies (HRP-GαM, Bio-Makor, Israel , for additional 30 min at 37°C. Determination of the amount of bound antibodies was obtained by washing cells and adding 100 µl of substrate (2,2′-azino-di(3-ethylbenzthiazolinesulphate) (Sigma) to each well. Absorption was recorded in a Titertech Multiskan MMC at 405 nm, with a reference wavelenght of 630 nm. Each bar represents an average (±SD) of 3 wells. The inset for comparison shows results obtained by counting the number of Galc positive cells per coverslip in cultures treated with either CM-R (12 µg/ml) or CM-N (12 µg/ml) added at 24 h in vitro and examined 48 h later.
**Figure 4** shows a comparison between PDGF and CM-R effects on newborn rat brain oligodendrocytes. Cultures of oligodendrocytes from neonatal rat brains were obtained as in Fig. 2. In all cultures, an identical number of cells was seeded. At 48 h in vitro, cultures were stained for either Galc or A₂B₅ positive cells. In treated cultures, either PDGF (5 ng/ml, Sigma or CM-R (12 µg protein/ml) were added at the time of seeding. As control, we used cultures kept in defined medium which were not treated. Fluorescent micrographs show cells stained with mouse A₂B₅ monoclonal antibodies in control cultures (b), cultures treated with PDGF (a), or cultures treated with CM-R (d). The bar graph in (c) shows the concrete counted number of A₂B₅ or Galc positive cells in the coverslips of each treatment (space bars = 10 µm).

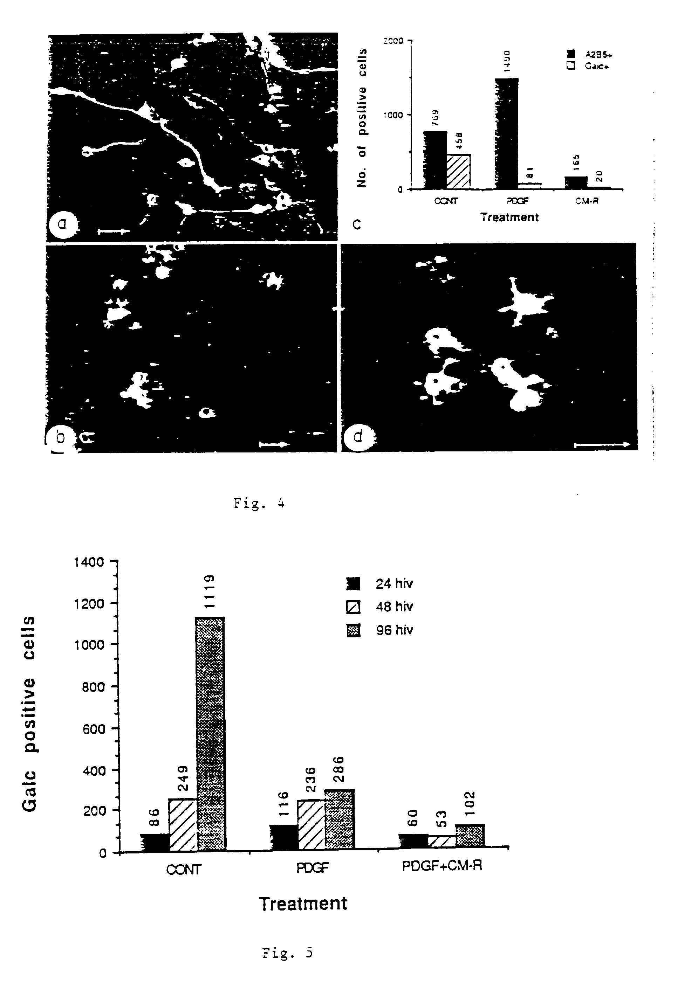

**Figure 5** shows the effect of combined application of CM-R and PDGF on development of Galc positive cells. Cultures of brain oligodendrocytes were prepared as in Figs. 2 and 3. PDGF (5 ng/ml) or PDGF together with CM-R (5 ng/ml and 12 µg/ml, respectively were added to neonatal rat brain oligodendrocytes 24 h after seeding. At the indicated subsequent time (24, 48, 96 h), cultures were stained for Galc positive cells. The numbers represent the absolute number of Galc positive cells per coverslip. Note the reduction in the number of Galc positive cells when treated with a combination of PDGF and CM-R.

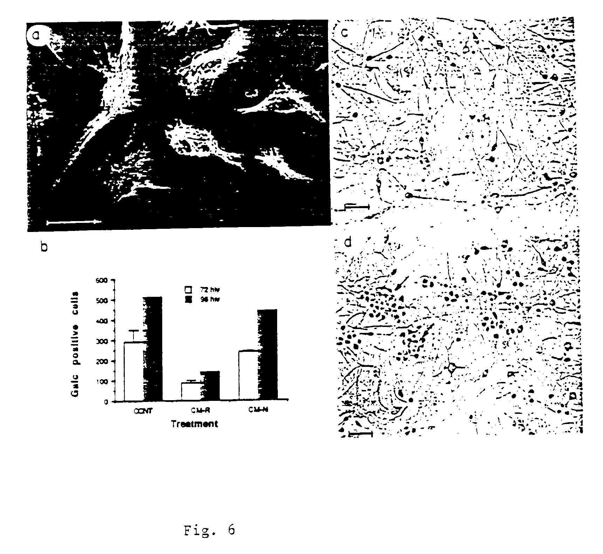

**Figure 6** shows that CM-R selectively affect oligodendrocytes in mixed newborn rat cultures of astrocytes and oligodendrocytes. Mixed glial cells were dissociated from newborn rat brains, as described in Fig. 2, and were immediately seeded on poly-L-lysine coated coverslips (10⁴ cell/coverslip). These cells were kept for 6 days in vitro in DMEM supplemented with 5% FCS, that was changed every 2 days. At day 6, the medium was changed into defined medium and supplemented with either CM-R (10 µg/ml) or CM-N (10 µg/ml). At 72 and 96 h in vitro, the cells were double labeled with mouse monoclonal antibodies against Galc and rabbit anti-GFAP, followed by specific rhodamine conjugated goat anti-mouse IgG₃ and fluorescein conjugated goat anti-rabbit, respectively. Fluorescent micrograph (a) shows cells stained with anti-GFAP and unaffected by CM-R treatment, after 72 h in vitro. Micrograph (b) shows the number of Galc positive cells in the coverslips of each treatment, at 72 and 96 h in vitro. The results represent mean±SD of 2 coverslips. Micrographs (c) and (d) are phase micrographs of typical mixed glial cells of untreated (d) and of CM-R treated cultures (c). Note that the monolayer formed by non-CM-R sensitive cells (c) are stained with GFAP. The CM-R sensitive cells in the control form clusters (d, marked by arrows) and are stained with A₂B₅ (data not shown). These cells represent probably the O-2A progenitors that are stimulated to proliferate by the monolayer forming type-1 astrocytes.

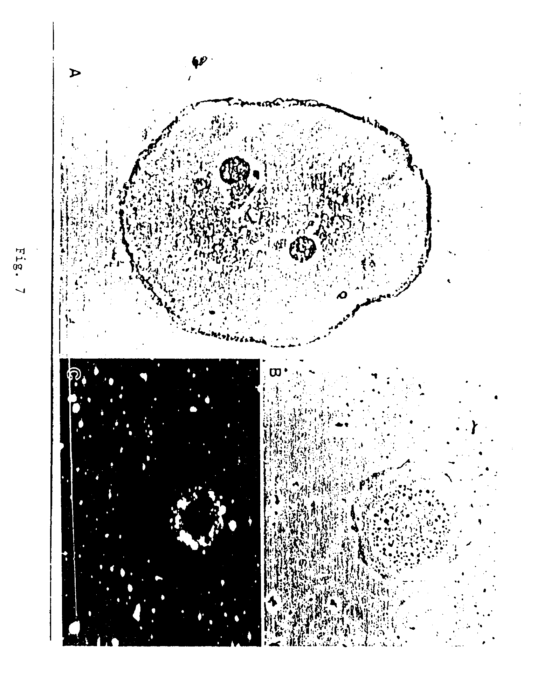

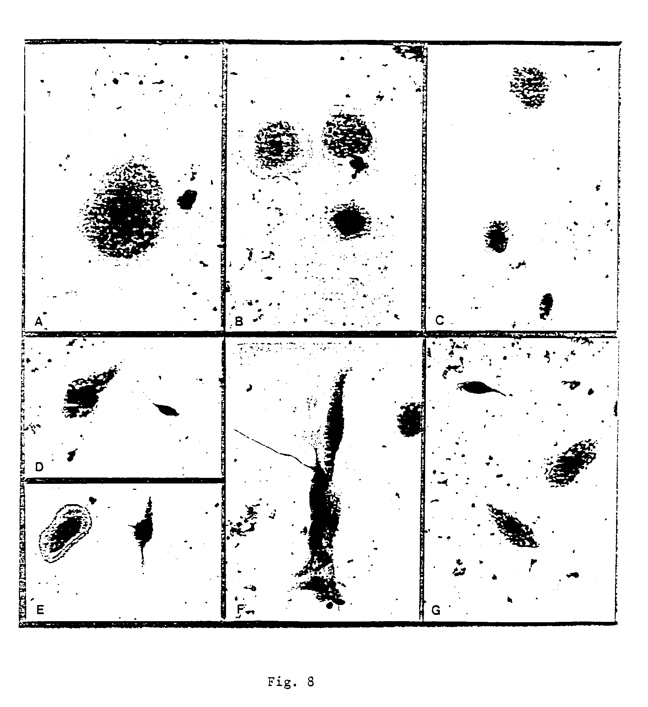

**Figure 7** shows blood-borne macrophages in the fish optic nerve cultures. These macrophages were most abundant in cultures of nerves which were injured several days prior to their excision. The cells seen here were grown in L-15 medium supplemented with 10% FCS. (A) shows a macrophage 7 days after plating, Giesma stained, and seen in phase contrast. (B) and (C) are cells 5 days after plating, (B) is phase contrast, and (C) is 6D2 labeled, after fixation. It is worth noting that (C) shows the myelin-loaded vacuoles of the macrophage. These vacuoles were also stained positively with nonspecific esterase (Fig. 8). All micrographs are 500x.
**Figure 8** shows macrophages positive for nonspecific esterase. Goldfish optic nerve cells were grown as described. (A), (B) and (C) show macrophages which are presumably blood-derived. These were rare in cultures of fish optic nerves which were organ cultured prior to their dissociation, but were abundant in cultures of fish optic nerves which were crushed a couple of days prior to their excision. These macrophages were typically circular in shape and their vesicles were also typically arranged in a circular fashion. These vesicles were 6D2-positive (Fig. 7 B,C), which is indicative of the myelin phagocytosis activity of these cells. Resident macrophages can be seen in (D) to (G). A variety of shapes was observed, ranging from long and fibroblast-like (F) to more round in appearance (D and G). The vesicles of these cells were evenly distributed throughout the cytoplasm, and were also 6D2-positive.

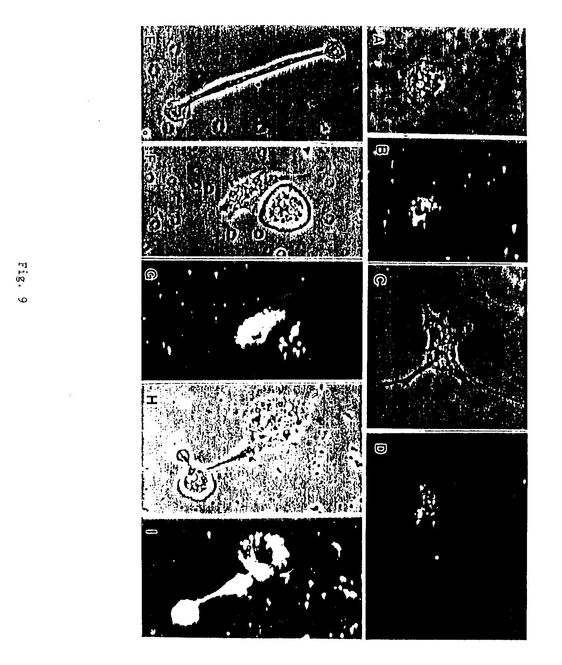

**Figure 9** shows resident macrophages in cultures of fish optic nerves which were organ cultured before dissociation. The figure shows different morphologies of the resident macrophages ranging from more round - (A) to (D) - to elongated, fibroblast-like appearance (E). Contact was observed between the resident macrophages and the blood-borne macrophages, as seen in (F) and (G). Contact was also observed between the oligodendrocytes and both types of macrophages, as seen in (H) and (I). The nature of this contact may be cell engulfing (H) and (I). (A), (C), (E), (F), and (H) are phase contrast, and all other micrographs are 6D2 labeled. All micrographs are 500x.

The novel oligodendrocyte cytotoxic factor (OCF) of the invention can be obtained from regenerating injured nerves of lower vertebrates, such as fish. It is present in the conditioned medium of regenerating fish optic nerves and can be isolated therefrom and further purified. It can also be isolated from macrophages, a much more available source, or any other suitable cellular source. It is isolated from the conditioned medium of said cellular sources and then purified.

The term "conditioned medium" (CM), as used throughout the present specification and claims, refers to a medium conditioned by regenerating fish optic nerves prepared by incubating segments of fish optic nerves, removed 8 days after crush, in a serum-free medium for 1.5- 3 h at room temperature, collected and filtered. The CM obtained is free of any tissue. Examples of serum-free media that can be used are Dulbecco's modified Eagle's medium (DMEM; 4 optic nerves per 300 µl medium), L-15 Leibowitz medium, etc. When comparison is made between conditioned medium of regenerating nerves and of intact nerves, then CM-R is used for CM of regenerating nerves and CM-N for CM of intact nerves.

The cytotoxic activity of OCF is measured by its ability to reduce the number of mature oligodendrocytes in rat brain cultures. The number of oligodendrocytes is determined by using antibodies directed to galactocerebroside (Galc), which label mature oligodendrocytes. Reduction in the number of Galc positive cells indicates reduction in the number of mature oligodendrocytes. The assay is disclosed with more details in the Experimental Procedures and Examples herein.

The cytotoxic activity of OCF is specific to oligodendrocyte lineage. Thus, it inhibits differentiation of progenitors of the oligodendrocyte lineage to mature oligodendrocytes. It has no cytotoxic effect on other cells, such as type-1 astrocytes or fibroblast cells.

OCF was found to be present in the CM of regenerating injured fish optic nerves, but not in the CM of non-injured fish optic nerves. It is also not present in the CM of injured mammal optic nerves. The OCF derived from regenerating injured nerves of fish reduces the number of mature oligodendrocytes in cultures of mammalian nerves, such as cultures of rat nerves.

OCF is water soluble and heat-sensitive, losing its activity at 56°C after 30 minutes. At 100°C, OCF loses its activity after 10 minutes.

OCF is sensitive to proteases, losing activity upon digestion with trypsin.

The factor of the invention is obtainable from the CM of regenerating fish optic nerves and then purified by conventional biochemical procedures, such as ion exchange chromatography, size exclusion chromatography, HPLC, etc.

The regeneration of CNS axons from fish optic nerves takes place in the presence of OCF, which is a factor derived from the environment of these injured nerves. OCF inhibits maturation of oligodendrocytes from their progenitors and causes a reduction in the number of process-bearing mature oligodendrocytes when added to a mammalian system. OCF was found to be active in rat cultures. Therefore, this factor may provide a way to avoid the repressive or nonpermissive effect of mature oligodendrocytes on regeneration in the adult mammalian CNS. Factors like OCF are not needed during development, as developing axons no not encounter fully differentiated myelin-producing oligodendrocytes, which are known to have a nonpermissive effect on axonal growth.

The observed effect of the substances derived from regenerating fish optic nerves on the number of cultured mammalian oligodendrocytes implies that the axonal growth in injured rabbit optic nerves treated with CM of regenerating fish optic nerves (Lavie et al., 1987) might be due to the possible in vivo effect ot these components in modulating the population of oligodendrocytes, in addition to their effect on the properties of astrocytes (Cohen and Schwartz, 1989). The observed inhibitory effect of OCF is not mediated via PDGF or CNTF, which indirectly cause a reduction in the number of mature oligodendrocytes (Noble et al., 1988; Lillien et al., 1988). Recent reports have implicated that activated astrocytes can be a source of factors which can inhibit oligodendrocyte maturation (Rosen et al., 1988) or for tumor necrosis factor (TNF) (Robbins et al., 1987), and that TNF has a cytotoxic activity on myelinated organ cultures of mouse spinal cord tissue (Selmaj and Ramie, 1988). Therefore, the possibility that the observed activity is mediated by a factor which is functionally similar to TNF was considered. To evaluate the relationship between the OCF of the present invention and TNF, antibodies directed to human recombinant TNF were used and shown not to neutralize the observed effect of CM-R.

Regeneration of the fish optic nerves takes place spontaneously after injury. Normal fish optic nerves can provide, in vitro, a permissive environment for mammalian CNS neurons to send out neurites. It is possible that the normal fish optic nerves are free of myelin-associated neuronal growth inhibitors, observed in mammals, or they have them but at a low level. If the fish have no or a low level of axonal growth inhibitors, it would imply that fish and mammals differ in their oligodendrocytes, or that inhibitory/cytotoxic factor(s) are regulating the expression of these myelin-associated axonal growth inhibitors. Such a possibility is compatible with the low levels of the inhibitory/cytotoxic factors found in the intact fish optic nerves. The fish visual system is unusual in that there is a continuous addition of cells throughout life and, thus, the optic nerve always contains a contingent of growing optic axons. Therefore, there is a possibility that a mechanism to grow in an adult environment is also present in the noninjured fish optic nerve, but to a much lesser degree. It is suggestive that mammals either miss the immediate posttraumatic accessibility to such factors at the appropriate time, or that they do not have these factors at all.

The cytotoxic effect found in the regenerating fish optic nerves might depend directly or indirectly upon macrophages, activated resident microglial cells, or activated astrocytes. If the macrophages rapidly invade the injured optic nerve of fish, as they do in regenerating peripheral nerves of mammals, they could be responsible for the observed increased activity in CM-R relative to CM-N, and for contributing to the elimination of the nonpermissive mature oligodendrocytes. This would create an environment permissive for growth while neurons are still in an injury-induced mode of growth.

It was observed that in cultures of nerves which were injured 2 days prior to their excision, many macrophages were present, but only few oligodendrocytes. In contrast, in cultures of uninjured optic nerves which have been organ cultured for 2 days prior to their dissociation, few macrophages were seen, but a relatively high number of oligodendrocytes. This inverse relationship between the number of oligodendrocytes and macrophages suggests a role for the macrophages in the regulation of the number of oligodendrocytes postinjury.

Monoclonal antibodies to OCF can be prepared by procedures well known in the art. Animals are immunized with a crude, enriched or purified preparation of OCF and the spleen and/or lymph node cells of the immunized animal are fused with suitable myeloma cells. Screening for positive hybridoma supernatants is performed with the bioassay for OCF described herein.

The monoclonal antibodies to OCF can be used for purification of OCF by affinity chromatography according to well-known procedures.

One of the ways of using the factor of the invention to induce regeneration of injured nerves of the CNS in mammals, consists in implanting a preparation of the factor in the target organ. For example, a longitudinal implant of the active soluble factor may be used throughout the nerve, to increase accessibility to the applied substance:minipump for continuous supply of the factor.

The invention will now be illustrated by the following experiments and examples which are presented in a non-limiting manner:

### Experimental Procedures

### a. Crush injury of fish optic nerve and preparation of CM (Conditioned Medium)

For preparation of 300 µl of CM, four carp (Cyprinus carpio, 800-1200 g) were deeply anesthetized with 0.05% tricaine methansulfonate (Sigma). The optic nerves were crushed intraorbitally and the animals replaced in their tank. Eight days later, the fish were reanesthetized, the crushed-regenerating nerves were dissected out, transferred into 300 µl of serum free medium (DMEM, Gibco) and incubated for 1.5 h at room temperature. Media were then collected, filtered and stored at -80°C. The protein content of the media was determined by the Bradford method (Bradford M., 1976).

Enzymatic digestion with trypsin was carried out as follows: Trypsin-linked to agarose was equilibrated with PBS and incubated for 4 h at 37°C with CM. The solution was centrifuged (1000 x g, 5 min); at the end of the incubation, the recovered supernatant was tested in vitro in the cultures described below. CM were heat treated at 100°C for 10 min.

### b. Crush injury of the rat optic nerve

Rats (250-350 g) were anesthetized with Rompum (10 mg/kg, intraperitoneally) and Vetalar (50 mg/kg, intraperitoneally). A lateral canthotomy was performed under a binocular operating microscope and the conjunctiva was incised laterally to the cornea, after separation of the retractor bulbi muscles. The optic nerve was identified and exposed near the eyeball by blunt dissection. The dura was left intact. The nerve was crushed using a calibrated cross-action forceps, 1 mm distal to the eye for a period of 30 sec. The canthotomy was sutured and the animal allowed to recover. At the appropriate time, the animals were reanesthetized and the optic nerves were excised.

### c. Preparation of cultures from injured adult rat optic nerves

To prepare the glial cell cultures, in each experiment, five adult rat optic nerves were used. The optic nerves were crushed as described above, and 3 days later they were excised, minced and incubated in Leibowitz L-15 medium (Gibco) containing 500 U/ml collagenase (Sigma). After 60 min at 37°C, an equal volume of trypsin (30000 U/ml) was added for additional 15 min. The suspension was centrifuged (500 x g, 5 min), the supernatant was removed and the tissue was then resuspended and further treated for 15 min with trypsin (15000 U/ml) in a solution containing EDTA 0.25 mM in Ca²⁺⁻ and Mg²⁺⁻ free DMEM. The digestion was terminated by addition of an equal volume of solution of soybean trypsin inhibitor (5000 U/ml, Sigma), bovine pancreas DNAse 1 (74 U/ml, Sigma), bovine serum albumin (BSA, fraction V, 3 mg/ml, Sigma), followed by centrifugation. The supernatant was replaced with Raff's modification (Raff et al., 1983) of Bottenstein's and Sato's defined medium (Bottenstein and Sato, 1978), followed by trituration of the tissue. Fifty µl of the resulting cell suspension was plated onto each poly-L-lysine (PLL, 20 µl/ml) coated coverslip (cells from one optic nerve were seeded on 3 coverslips). After 30 min at 37°C, the adherent cells were rinsed and 500 µl of defined medium was added. CM from regenerating fish optic nerves (12 µm/ml) were added to experimental samples 48 h later. The cells were examined after additional 48 h, by indirect immunofluorescence labeling.

### d. Cell cultures from newborn rat brain

Glial cells were prepared from cerebral cortex of newborn Sprague-Dawley rats (McCarthy and DeVellis, 1980). Cells were plated into PLL-coated flasks (85 mm², Nunc) or onto PLL-coated coverslips (10⁵ cells/coverslip), for analysis of mixed glial cell cultures. The cells were grown in DMEM supplemented with 5% fetal bovine serum (FBS, Sigma), that was changed every 2 days.

### e. Preparation of enriched oligodendrocyte cultures

After 8 days in vitro, the flask containing mixed populations of glial cells of newborn rat brains was shaken overnight, the nonadherent cells were plated onto PLL-coated coverslips at approximately 10⁴ cells/coverslip, unless otherwise specified. The cells were allowed to adhere for 30 min and were then fed with Raff's modification of Bottenstein and Sato's defined medium, in order to encourage oligodendrocyte development. Cells were treated at different time periods after seeding and were subjected to indirect-immunofluorescence staining or ELISA, usually after 48 h, unless otherwise specified.

### f. Indirect immunofluorescence labeling

Cultured cells were immunolabeled with the following antibodies: A₂B₅ mouse IgM monoclonal antibodies (hybridoma supernatant, diluted 1:1), which label mature type-2 astrocytes and perinatal progenitors of both oligodendrocyte and type-2 astrocyte (O-2A perinatal progenitors) (Eisenbarth et al., 1979; Raff et al., 1983); O4 mouse IgM monoclonal antibodies (concentrated hybridoma supernatant, diluted 1:100), which label immature oligodendrocytes (Sommer and Shachner, 1981) and O-2A adult progenitors (ffrench-Constant and Raff, 1986a); mouse antigalactocerebroside (Galc) monoclonal antibodies (hybridoma supernatant, diluted 1:10), which label mature oligodendrocytes (Raff et al., 1978); rabbit anti-glial fibrillary acidic protein (GFAP, whole sera, diluted 1:1000), which label mature type-1 and type-2 astrocytes (Bignami et al., 1972); mouse monoclonal antibodies 6D2 which label fish oligodendrocytes (Jeserich and Romen, 1989). Rhodamine- or fluorescein-conjugated second antibodies were purchased from different sources: goat anti-mouse IgM (diluted 1:50, Jackson Immunoresearch Laboratories); goat anti-mouse IgG₃ (diluted 1:50, Serotec); swine anti-rabbit IgG (diluted 1:50, Dakopatts).

Immunolabeling of surface antigens (A₂B₅, O4 and Galc) was carried out first by incubating each of the required antibodies with the cells in 50 µl volume at 37°C for 30 min. Cells were then washed several times with Hank's balanced salt solution containing 2% heat-inactivated FBS, and further incubated for 30 min in the appropriate rhodamine- or fluorescein-conjugated second antibodies diluted in DMEM. The cells were washed and fixed in methanol at -20°C, for 10 min. For intracellular antigens (GFAP), cells were first fixed and then immunolabeled. In some experiments, cultures were double labeled simultaneously with A₂B₅ and Galc antibodies, followed by anti-mouse IgM (rhodamine) and anti-mouse IgG₃ (fluorescein). In all cases, after the immunolabeling, the coverslips were washed, mounted in a drop of glycerol containint 22 mM of 1,4-diazobicyclo(2,2)octaine (Sigma), to prevent fading, sealed and examined in a Zeiss Universal microscope equipped with phase contrast, epifluorescence (for rhodamine and fluorescein detection) optics and camera. The concrete number of all immunolabeled cells were counted in all coverslips.

### g. ELISA on living cells

Cultures enriched for oligodendrocytes were prepared as described above. The cells were seeded in multiwell microtiter plates (5 x 10³ cells/well, Nunc). After 24 h in vitro, CM derived from either regenerating nerves or intact nerves were added at the indicated concentrations. Forty-eight hours later the cells were examined by incubating with Galc antibodies at 37°C for 30 min, followed by horseradish peroxidase conjugated to goat anti-mouse antibodies (BioMakor, Israel), for additional 30 min at 37°C. Determination of the amount of bound antibodies was obtained by washing the cells and adding 100 µl of substrate [2,2-azino-di(3-ethylbenzthiazoline)sulphate, Sigma], supplemented with 0.003% H₂O₂ to each well. Absorption was recorded in a Titertech Multiskan MMC at 405 nm, with a reference wavelength of 630 nm.

### h. Nonspecific Esterase Staining

Cells were stained using the α-Naphthyl Acetate Esterase procedure, as well as the fluoride inhibition procedure using a Sigma kit. Sodium nitrate (50 µl; 0.1 M) was added to 50 µl fast-blue (15 mg/ml in 0.4 M HCl). The mixture was left for 2-3 minutes at room temperature. To this, 2 ml deionized water at 37 °C was added, followed by 250 µl Trizmal 7.6 buffer concentrate, followed by 50 µl α-Naphthyl Acetate solution. For fluoride inhibition, 50 µl sodium fluoride (0.2 g/ml) was also added. Cells were then fixed in a citrate-acetone-formaldehyde fixative (25 ml citrate solution plus 65 ml acetone plus 8 ml of 37% formaldehyde) for 30 sec at room temperature. The cells were then washed thoroughly in water, after which they were incubated for 30 min with the α-Naphthyl Acetate reaction solution at 37°C. After being thoroughly washed, the cells were counterstained for 2 min with Hematoxylin solution. The coverslips were then washed and mounted on microscope slides with glycerol for observation.

### i. Statistical analysis

The statistical analysis of the results was performed using the Friedman Rank Test. In this statistical test each experiment is treated as a separate block, and the significant difference of the results (control vs CM-treated) is found, according to the number of blocks.

### EXAMPLES

### Example 1

This example shows that media conditioned by regenerating fish optic nerves containing OCF cause reduction in process-bearing oligodendrocytes in cultures of dissociated injured adult rat optic nerves.

Fig. 1a,b shows process-bearing 04 positive cells in cultures derived from adult rat optic nerves, which had been injured 3 days prior to their removal. In the presence of conditioned media (CM) derived from regenerating fish optic nerves (CM-R;12 µg protein/ml) added to these cultures 48 h after seeding, the number of 04 positive cells that appeared at 96 h in vitro (Fig. 1c-g) was much lower than in non-treated cultures kept in the control medium (Fig. 1a,b) The 04 positive cells which remained in the CM-R treated cultures had few processes and were therefore, identified as immature oligodendrocytes (Fig. 1c-g). The same results were obtained when the same experiment was repeated using antibodies directed to mature oligodendrocytes, such as antigalactocerebroside (Galc) rather than 04 antibodies. Accordingly, in cultures treated with CM of fish regenerating nerves (CM-R), only 42% of the cells developed into mature Galc positive cells out of the total Galc positive cells in the defined medium. These results suggest the presence of soluble factor(s) in media conditioned by regenerating fish optic nerve, which may be cytotoxic and/or may inhibit maturation of oligodendrocytes from their progenitors.

### Example 2

This example shows that the media conditioned by regenerating fish optic nerves containing OCF affect maturation of Galc positive cells in newborn rat cultures of oligodendrocytes.

In order to further study the nature of this inhibitor/cytctoxic factor and its possible implication for regeneration, we first looked for a richer source of oligodendrocytes and examined the possibility of using cultures of newborn rat brains instead of injured adult rat optic nerves, as they provide a rich source and a more homogeneous population of oligodendrocytes.

Newborn rat brain oligodendrocytes and their perinatal progenitors were obtained in the following set of experiments by the method of McCarthy and DeVellis, 1980. The oligodendrocytes were seeded on poly-L-lysine coated coverslips. CM of regenerating fish optic nerves (CM-R) were added to these cultures (12 µg/ml) enriched with newborn rat brain oligodendrocytes either at the time of seeding or 24 h and 48 h later. In all cases, the same number of oligodendrocytes were seeded as in control untreated cultures. As can be seen in Figure 2, in all CM-treated cultures 24 h subsequent to the CM application, the number of multiprocessed Galc positive cells was lower (about 50%) than in matched non-treated cultures. It appears that if the CM affects only maturation of oligodendrocytes from their progenitors, the low number of Galc positive cells observed, at least in cultures treated 24 h or 48 h after seeding, might be a reflection of spontaneous cell death under conditions in which cell renewal from progenitors is inhibited. Alternatively, the CM of the regenerating fish optic nerve might affect mature oligodendrocytes in addition to its effect on progenitors. Also shown in Figure 2, is the dependency of the inhibitory effect on the amount of the applied CM. Thus, while 12 µg protein/ml were effective, 1.2 µm protein/ml were ineffective. As shown in Table I, 24 h after CM-R application, the number of process-bearing Galc positive cells was already lower by 40.3 ± 6.4, relative to their number in untreated control cultures (Table I). Boiling the CM-R or treating it with trypsin resulted in a diminution of their inhibitory/cytotoxic effect (Table II), thus suggesting that the factor has a protein nature.

**Table I**

| *CM-R cause a reduction in the number of rat brain oligodendrocytes* | | | |
|---|---|---|---|
| Type of * cells | Hours ** of post-treatment | % of control*** (mean ± SD) | Number of experiments (n) |
| Oligodendrocytes | 24 | 40.3± 6.4 | 3 |
| Oligodendrocytes | 48 | 14.4±10.4 | 12 |
| Mixed glial cells | 48 | 25.7± 4.7 | 4 |

| | | | |
|---|---|---|---|
| * In these experiments, the number of counted oligodendrocytes (Galc positive cells) in control cultures per coverslip was between 350-1700 cells. | | | |
| ** CM-R at 12 µg protein.ml was added. | | | |
| *** The percentage of inhibition was calculated in each experiment by taking the number of counted cells in control, nontreated cultures, as 100%. | | | |

**Table II**

| *The inhibitory/cytotoxic effect of CM-R on oligodendrocytes is heat and trypsin labile* | |
|---|---|
| Treatment | Number of Galc positive cells (mean ± SEM) |
| Control | 1427±247 |
| CM-R | 361± 17 * |
| CM-R (boiled) | 1474± 70 ** |
| CM-R (trypsin treated) | 1087± 10 ** |
| In this experiment, rat brain oligodendrocytes were cultured as in Fig. 2. At 24 h after plating, CM-R (boiled or trypsin treated, 20 µg/ml) was added. Forty-eight hours after the addition of CM-R, the number of Galc positive process-bearing cells was determined by counting immunofluorescent cells. The results of one experiment, carried out in triplicate, are given. These results were reproduced in two additional experiments. | |

| | |
|---|---|
| * p-value = 0.025. | |
| ** No significant differences were observed between control cultures and cultures treated with boiled or trypsinized CM-R. | |

### Example 3

This experiment shows that the inhibitory/cytotoxic activity of OCF is associated with regeneration in the fish optic nerves.

In order to find out whether the level or activity of the inhibitory/cytotoxic factor are correlated with regeneration of the fish optic nerves, we compared the effect of the CM-R with that of media conditioned by non-injured normal fish optic nerves (CM-N). CM-R or CM-N, at the same protein concentrations, were added to newborn rat brain oligodendrocytes 24 h after seeding. The resultant effect was monitored with anti-Galc antibodies, using counts of Galc positive cells labeled by indirect immunofluorescence. Fig. 3 compares the number of Galc positive cells in CM-N treated cultures relative to their number in CM-R treated culture. The effect of CM-N was lower i.e., non-injured nerves lacked inhibitory activity on maturation of oligodendrocytes.

### Example 4

This experiment shows that OCF comprised in CM-R does not mimic the effect of PDGF.

Differentiation of oligodendrocytes has recently been shown to be regulated by platelet-derived growth factor (PDGF) (Noble et al., 1988, Raff et al., 1988 and Richardson et al 1988). Addition of PDGF to cultures of oligodendrocyte progenitors transiently delays their differentiation, due to its mitogen activity.

To get an insight as to the possible mechanism underlying the observed inhibition, at least that related to the maturation of oligodendrocytes from their progenitors, we compared activity of CM of regenerating fish optic nerves (CM-R) to that of PDGF. Addition of PDGF to brain cultures at the time of seeding, resulted in an apparent reduction in the number of Galc-positive cells at 48 h in vitro. However, the effect was markedly less as compared with that of the CM-R (Fig. 4). The apparently lower number of Galc positive cells observed in PDGF-treated cultures relative to control non-treated cells was a reflection of the proliferation of their progenitors, stained with A₂B₅ antibodies. This was evident from the increased number of A₂B₅-positive cells in PDGF-treated cultures. As can be seen in Figure 4, in PDGF-treated cultures, as expected, there was an increase in the number of 0-2A progenitors, stained with A₂B₅ antibodies (Fig. 4a, b, c). In contrast, in CM-treated cultures there was a reduction in the number of A₂B₅ positive cells (Fig. 4c, d). These results suggest that CM-R affect not only adult oligodendrocytes, but also their progenitors. These observations rule out the possibility that the observed effect of the CM-R on the development of Galc positive cells from their progenitors is mediated by PDGF or any other mitogen. This is further supported by Figure 5, which shows that addition of CM-R together with PDGF resulted in a reduction of Galc positive cells, as would be expected from the application of CM-R only.

Ciliary neurotrophic factor (CNTF) is another factor which has recently been shown to be effective in O-2A cell lineage by inducing type-2 astrocyte differentiation and thereby indirectly causing a reduction in the number of mature Galc positive oligodentrocytes Lillien et al., 1988). Since the CM-R did not cause an increase in the number of cells stained with A₂B₅ antibodies, and thus did not increase either progenitors or type-2 astrocytes, it is unlikely that the CM-R effect is mediated via CNTF.

### Example 5

This experiment shows the specificity of the inhibitory/cytotoxic effect of OCF on oligodendrocytes by media conditioned with regenerating fish optic nerves.

In order to examine whether the observed inhibitory/cytotoxic effect of the CM is unique to oligodendrocytes, we applied the CM-R to a mixed population of astrocytes and oligodendrocytes.

Figure 6 shows that the observed inhibitory effect is not due to a non-specific toxic effect but is rather selective to oligodendrocytes. Cultures of mixed population of glial cells, dissociated from newborn rat brain, were treated with either CM of regenerating, or of intact nerves (CM-R and CM-N, respectively). Such conditions are the most favorable for differentiation of oligodendrocytes seeded onto the monolayer by the astrocytes. Even under these conditions, the inhibitory effect of the CM-R was pronounced, whereas the CM-N had only a marginally inhibitory activity at 72 and 96 h in vitro (Fig. 6b). In contrast to the inhibitory effect of the regenerative CM on oligodendrocytes, the survival and differentiation of non-oligodendrocyte cells, which were present in the these mixed cultures and stained with glial fibrillary acidic protein (a marker for astrocytes) were not affected by the CM-R (Fig. 6a). The apparent density of the monolayer of astrocytes formed in treated and non-treated cultures was similar (Fig. 6c vs. Fig. 6d). The observed inhibitory effect of the CM-R is therefore not due to a non specific toxic effect, but is rather selective to oligodendrocyte lineage (Fig. 6).

### Example 6

These experiments show that there is an inverse relationship between the numbers of oligodendrocytes and macrophages.

In the first experiment, common goldfish were used. The conditioned media was prepared with Dulbecco's modified Eagle's medium (DMEM, 4 optic nerves per 300 µl/medium).

Comparison between the cell populations obtained after dissociation of nerves which were kept in organ culture and those of the regularly crushed nerves (i.e., OC-3 and PC-3 in rat and OC-2 and PC-2 in fish) might indicate whether the postinjury behaviour of these cells is intrinsic to the optic nerve, or whether it is affected by some external factor, such as the blood monocytes. Any such external factors would be absent in the organ cultures, since the optic nerve is taken out at the time of injury.

In cultures of dissociated fish optic nerves which were organ cultured for 2 days prior to dissociation (OC-2 cultures), a relatively high number (65 cells per coverslip) of oligodendrocytes (6D2 positive cells) was observed, but only few macrophages were seen. In contrast, in cultures of dissociated optic nerves which were excised 2 days after their lesion (PC-2 cultures), many macrophages developed, but only few oligodendrocytes. The few oligodendrocytes which were seen in cultures of crushed nerves (PC-2) were much smaller, and had much shorter processes than the oligodendrocytes in the OC-2 cultures. These results are summarized in Table III.

In parallel to the appearance of the oligodendrocytes in the dissociated cells of the organ cultured nerves, there was a complete absence of macrophages or macrophage-like cells in these cultures. Even after 7 days in culture none of these cells were seen (by this time, in PC-2 cultures these cells reach an enormous size).

**Table III**

| The number of oligodendrocytes in dissociated cultures of fish optic nerves which had been crushed 2 days prior to excision and dissociation (PC-2 cultures), and in cultures of fish optic nerves which had been organ cultured for 2 days prior to dissociation (OC-2 cultures)¹ | | | | |
|---|---|---|---|---|
| Culture | No. of oligodendrocytes | | | |
| | Exp.1 | Exp.2 | Exp.3 | Mean±SD |
| OC-2 | 65(±3) | 93(±25) | 40(±12) | 66(±26) |
| PC-2 | 3(±1) | 2(±1) | 15(±5) | 7(±6) |

| | | | | |
|---|---|---|---|---|
| ¹ Within each experiment, the same number of fish, of approximately equal size, was taken for the PC-2 and for the OC-2 cultures. Also, the cells were finally seeded on an equal number of coverslips. In this way, variations due to the experimental procedure were reduced to the minimum. Each experiment was carried out in triplicate. The total number of oligodendrocytes (6D2 positive cells) on the coverslips was counted, and the numbers given are the averages of three trials. | | | | |

Unlike the situation in the fish, in the rat the number of mature oligodendrocytes that was observed in cultures of injured nerves and of organ cultured nerves was similar. In cultures of rat optic nerves, which were organ cultured for 3 days (OC-3 cultures), three main populations of cells were observed. Similarly to cultures of crushed rat optic nerves (Fig. 3), a large proportion of these cultures were process-bearing oligodendrocytes which were labeled by Galc after 96 h in culture, and comprised nearly 40% of the total cell number. Another large population of cells (about 40%) morphologically resembled macrophages.

In the following experiments, goldfish optic nerve cells were grown either in L-15 Leibowitz medium supplemented with 1-2% FCS, or in L-15 medium supplemented with the same additives used in Raff's modification of Bottenstein and Sato defined medium, plus 1-2% FCS. Organ culture of fish optic nerves was performed in L-15 medium supplemented with the same additives used in Raff's modification of Bottenstein and Sato defined medium, plus 1-2% FCS.

Medium conditioned by regenerating goldfish optic nerves (CM) was prepared similarly to the CM-R determined above by incubating segments of goldfish optic nerves, removed 8 days after crush, in L-15 medium (10 goldfish optic nerves per 500 µl of medium for 3 h at room temperature).

The macrophages in the fish cultures included blood-derived as well as resident macrophages. The two types of macrophages were distinguishable by morphological criteria: the blood-derived macrophages were typically circular and their vesicles were also arranged in a circular manner; the resident macrophages, on the other hand, were irregular in shape and their vesicles were distributed randomly through the cytoplasm. Moreover, cells which were reminiscent of the blood-derived macrophages (Fig. 7) were abundant in cultures of optic nerves excised several days after a crush injury, but were rare in cultures of optic nerves organ cultured after the excision. Both macrophage types actively phagocytozed myelin debris, as evidenced by the 6D2-labeled vacuoles on prefixed cells (the vacuoles did not label by any other antibody used, nor by the second antibody itself). The apparently blood-derived macrophages grew best when the medium was supplemented with 10% FCS, and after a week in culture they grew to very large dimentions, compared to the other cells in the culture (Fig. 7A). Fig. 7C shows a macrophage whose vacuoles are heavily labeled by the 6D2 antibody. These macrophages were also positively stained with nonspecific esterase (Fig. 8A-C).

The cells which were considered as resident macrophages appeared in many forms, but could be divided into two major groups: those with a straight, fibroblast-like appearance (Fig. 8F and Fig. 9E), and those which were rounder and thicker, and which sometimes possessed processes (Fig. 9A-D). Contact was observed between the blood-derived macrophages and the resident macrophages (Fig. 9F-G), and between both macrophage types and the oligodendrocytes (Fig. 9H-I). The nature of this contact seems to be cell-engulfing; oligodendrocytes seem to be engulfed by both macrophage types. These resident macrophages were also nonspecific esterase positive (Fig. 8D-G).

In view of the inverse relationship between the numbers of oligodendrocytes and macrophages, and the effect the CM has on rat oligodendrocytes, we examined whether the soluble substances derived from regenerating fish optic nerves (i.e. -CM) are responsible for the postinjury oligodendrocyte regulation. When the CM was added to cultures of dissociated optic nerves, which had been organ cultured for 2-3 days, the number of oligodendrocytes decreased. Because of the low number of oligodendrocytes per coverslip, the experiment was repeated several times. The statistical test showed that the effect of the CM on the oligodendrocyte number is significant. These results are shown in Table IV.

**Table IV**

| The effect of medium, conditioned by regenerating fish optic nerves (CM), on the number of oligodendrocytes in fish optic nerve cultures ^{a}. | | | | |
|---|---|---|---|---|
| Experiment no.^{b} | Number of oligodendrocytes ^{c} (±S.D.)^{d} | | | |
| | *Control* | | *CM - treated* ^{e} | |
| 1 | 24.5 | ±2.0 | 13 | ±1.5 |
| 2 | 57 | ±16 | 30 | ±3.5 |
| 3 | 36 | ±8.0 | 26 | ±12 |
| 4 | 24 | ±4.0 | 19 | ±4.0 |
| 5 | 19 | ±3.0 | 3.5 | ±2.5 |
| 6 | 13.5 | ±0.6 | 4.5 | ±0.6 |
| 7 | 15 | ±3.5 | 7 | ±5.0 |

| | | | | |
|---|---|---|---|---|
| ^{a} Optic nerves were excised and organ cultured for two days. The optic nerves were then dissociated and plated. At this stage the conditioned media of the regenerating fish optic nerves (CM) was added to the appropriate wells. | | | | |
| ^{b} Because of the small cell number, the experiment was repeated several times. | | | | |
| ^{c} Each experiment was done in triplicate, the mean of which is given. | | | | |
| ^{d} Standard deviation of the triplicates. | | | | |
| ^{e} The CM was added to give a final concentration of 40 µg.ml. The Friedmann Rank Test used to statistically analyze the results gave a value of p = 0.0001 for the significant difference between the control and CM-treated coverslips. | | | | |

The Friedman block rank test, which was used to analyze the results, showed that the significant difference between the control (untreated) and CM treated coverslips was equal to 0.01% (p = 0.0001). When medium conditioned by non-regenerating (normal) goldfish optic nerves (CMN) was similarly added to the cultures, no effect was seen on the number of oligodendrocytes, as shown in Table V.

**Table V**

| The effect of medium conditioned by normal fish optic nerves (CMN) on the number of fish oligodendrocytes, compared to medium conditioned by regenerating fish optic nerves (CM) ^{a}. | | | | | |
|---|---|---|---|---|---|
| **Number of oligodendrocytes (±S.D.)** ^{**b**} | | | | | |
| *Control* | | *CM - treated* ^{e} | | *CMN - treated* ^{e} | |
| 92 | ±22 ^{c} | 50 | ±6 ^{c} | 103 | ±15 ^{c} |
| 46 | ^{d} | ND | | 45 | ^{d} |

| | | | | | |
|---|---|---|---|---|---|
| ^{a} The experiment was carried out as described in table 1. | | | | | |
| ^{b} Standard deviation of triplicates. | | | | | |
| ^{c} Each experiment was done in triplicate, the mean of which is given. | | | | | |
| ^{d} Experiment was carried out in duplicates. Therefore, no SD is given. | | | | | |
| ^{e} The CM and CMN were added to give a final concentration of 40 µg/ml. | | | | | |

### Example 7

This experiment shows the sensitivity of the OCF of the invention to treatment at 56°C.

Determination of Galc positive cells in cultures of newborn rat brain cells enriched for oligodendrocytes is carried out by ELISA as described in Experimental Procedures. As can be seen from Table VI, at 56°C there is a reduction in the number of Galc positive cells as evidenced by the reduction in the measured optical density.

**Table VI**

| Temperature sensitivity of the OCF comprised in the conditioned medium (CM) as measured by ELISA | | |
|---|---|---|
| Concentration^{a} | Average optical density ±SD | |
| | Room Temperature | 56°C^{b} |
| - | 0.752±0.076^{c} | - |
| CM(200 µg/ml) | 0.556±0.008 | 0.738±0.040 |
| CM(20 µg/ml) | 0.646±0.029 | 0.709±0.016 |

| | | |
|---|---|---|
| ^{a} Final concentration of the CM | | |
| ^{b} The culture is heated at 56°C for 30 min. | | |
| ^{c} Experiment done in 4 trials, the mean of which is given. | | |

### Example 8

This experiment shows the effect of conditioned medium of fish blood macrophage on oligodendrocytes.

A macrophage-rich culture was prepared as follows. Blood was collected from fish into PBS containing heparin, and then layered over Hypaque-Ficoll gradient made by mixing 70.6 ml of 12% w/v Ficoll (Sigma) with 29.4 ml of 32.8 w/v sodium diatriozate (Sigma). Ten ml of blood were layered over 5 ml of Hypaque-Ficoll in a 15 ml polystyrene tube. The tubes were centrifuged at 1000 g for 25 min at 10°C. The buffy coat containing lymphocytes and monocytes were removed and washed once with PBS. Cells were collected and adjusted to 20 x 10⁶ cells/ml. After 1 h at 16°C, the cells were washed 3 times with PBS to remove loosely adherent and non-adherent cells. Then L-15 medium was added to the cells.

Medium conditioned by macrophage-rich culture was prepared by incubating the cells in a serum-free medium for 8 h or in medium containing lipopolysaccharide. This conditioned medium was added to oligodendrocyte cultures and the number of oligcdendrocytes was determined by counting immunofluorescent Galc positive cells, as described in Experimental Procedures. In the first experiment, the number of Galc-positive cells counted in the presence of the conditioned medium of the macrophage-rich culture was 420 in comparison to 839 in the control. In the second experiment, there were 24 Galc-positive cells in comparison to 62 in the control.

### Example 9

### Pharmaceutical compositions

The oligodendrocyte cytotoxic factor OCF of the invention is useful as active ingredient of pharmaceutical compositions to be used for regeneration of nerves of the central nervous system. Preferably, it will be administered locally to the target organ.

The OCF is used in a quantity and purity sufficient to facilitate regeneration of CNS axons in mammals, particularly humans. The OCF is administered in any manner which is calculated to bring the factor to the vicinity of the injured axons to be regenerated. Preferably, the OCF is injected in a pharmaceutically acceptable liquid carrier directly to the site. Alternatively, an implant bearing the OCF may be surgically inserted. Such an implant may consist of any material, such as nitrocellulose, which will absorb OCF like a sponge and slowly release it at site of implantation. Other means of delivery will be apparent to those skilled in this art, and are intended to be comprehended within the scope of the present invention.

The amount of the OCF to be administered to any given patient depends on a variety of factors, such as the injury being treated, the site of injured axons it is wished to regenerate and the condition of the patient. Typically, however, the OCF is administered at a dose of about 100 units, as a single injection or as multiple injections around the injured site, or the OCF is soaked onto nitrocellulose or any other adsorbable carrier. Precise dosages will be empirically determined.

The OCF is preferably administered as soon as possible after the injury being treated. Thus, it is preferably used for acute injury rather than chronic injury. It will be more difficult to facilitate regeneration in accordance with the present invention the longer a period of degeneration has existed.

While the administration of OCF alone shows good results, such treatment may be combined with any type of concomitant theraphy which may tend to augment its effects. For example, irradiation of the injury site with low energy laser, preferably He-Ne laser (5 min/day, 35 mW) can delay the post-traumatic process of the degeneration and thereby delay scar formation (Assia et al., Brain Res., **476**, 205-212 (1988).

The various injuries which can be treated in accordance with the present invention are myriad and will be readily apparent to those of ordinary skill in the art. Without limitation, there may be mentioned spinal cord injuries, injuries to the optical nerve or to the aural nerves, etc. Injury to CNS neurons during neurosurgery or caused by tumors may also be treated by means of the present invention.

For the purpose of the present invention, the OCF may be formulated with any pharmaceutically acceptable carrier or excipient. The OCF may be presented as an aqueous solution, for example as a sterile aqueous solution. A solution or powder containing OCF may be stabilized by means of a stabilizing agent. The composition is administered to the site of the injury in a quantity effective for facilitation of the regeneration of injured axons.

### References

1. Bignami, A., Eng, L.F., Dahl, D. and Uyeda, C.T., Localization of glial fibrillary acidic protein in astrocytes by immunofluorescence, Brain Research, **43** (1972) 429-433.
2. Bottenstein, J.E. and Sato, G.H., Growth of a rat neuroblastoma cell line in serum-free supplemented medium, Proc.Natl.Acad.Sci.USA, **76** (1978) 514-517
3. Bradford, M., A rapid sensitive method for quantitation of microgram quantities of protein utilizing the principle of protein-dye binding, Anal.Biochem. **72** (1976) 248-256.
4. Cohen, A. and Schwartz, M., Conditioned media of regenerating fish optic nerves modulate laminin levels in glial cells, J.Neurosci.Res., **22** (1989) 269-273.
5. Eisenbarth, G.S., Walsh, F.S. and Nirenberg, M., Monoclonal antibody to a plasma membrane antigen of neurons, Proc.Natl.Acad.Sci.USA, **76** (1979)4913-4917.
6. ffrench-Constant, C. and Raff, M.C., Proliferating bipotential glial progenitor cells in adult rat optic nerve, Nature (Lond.), **319** (1986a) 499-502.
7. Hadani, M., Harel, A., Solomon, A., Belkin, M., Lavie, V. and Schwartz, M., Substances originating from optic nerves of neonatal rabbit induce regeneration-associated response in injured nerves of adult rabbit, Proc.Natl.Acad.Sci.USA, **81** (1984) 7965-7969.
8. Jeserich, G. and Romen, T., Glia **3 (1)** (1989) 65-74.
9. Lavie, V., Harel, A., Doron, A., Solomon, A., Lobel, D., Belkin, M., Ben-Bassat, S., Sharma, S. and Schwartz, M., Morphological response of injured adult rabbit optic nerves to implants containing media conditioned by growing optic nerves, Brain Research, **419** (1987) 166-172.
10. Lillien, L.E., Sendtner, M., Rohrer, H., Hughes, S.M. and Raff, M.C., Type-2 astrocyte development in rat brain cultures is initiated by a CNTF-like protein produced by type-1 astrocytes, Neuron, **1** (1988) 485-494.
11. McCarthy, K.D. and DeVellis, J., Preparation of separate astroglial and oligodendroglial cell cultures from rat cerebral tissue, J.Cell.Biol. **851** (1980) 890-902.
12. Miller, R.H. and Raff, M.C., J.Neurosci. **4** (1984) 585-592.
13 Noble, M. Murray, K., Stroobant, P., Waterfield, M.D. and Riddle, P., Platelet-derived growth factor promotes division and motility and inhibits premature differentiation of the oligodendrocyte/type 2 astrocyte progenitor cell, Nature(Lond.) **333** (1988) 560-562.
14. Raff, M.C., Lillien, L.E., Richardson, W.D., Burne, J.F. and Noble, M.D., Platelet-derived growth factor from astrocytes drives the clock that times oligodendrocyte development in culture, Nature(Lond.) **333** (1988) 562-565.
14. Raff, M.C. and Miller, R.H., T.I.N.S. **7** (1984) 469-472.
16. Raff, M.C., Miller, R.H. and Noble, M., A glail progenitor cell that develops in vitro into an astrocyte or an oligodendrocyte depending on the culture medium, Nature(Lond.), **303** (1983) 390-396.
17. Raff, M.C., Mirsky, R., Fields, K.L., Lisak, R.P., Dorfman, S.H., Silberberg, D.H., Grenson, N.A. Leibowitz, S. and Kennedy, M.C., Galactocerebroside is a specific cell-surface antigenic marker for oligodendrocytes in culture, Nature(London), **274** (1978) 813-816.
18. Robbins, D.S., Shirazi, Y., Drysdale, B., Liberman, A., Shin, H.S. and Shin, M.L., Production of cytotoxic factor for oligodendrocytes by stimulated astrocytes, J.Immunol., **139** (1987) 2593-2597.
19. Rosen, C.L., Bunge, R.P., Ard, M.D. and Wood, P.M., Type 1 astrocytes inhibit myelination by adult rat oligodendrocytes in vitro, J.Neurosci., **9** (1988) 3371-3379.
20. Richardson, W.D., Pringle, N., Mosley, M.J. Westermark, B. and Dubois-Dalcg, M., A role for platelet-derived growth factor in normal gliogenesis in the central nervous system, Cell, **53** (1988) 309-319.
21. Schwartz, M., Belkin, M., Harel, A., Solomon, A., Lavie, V., Hadani, M., Rachailovich, I. and Stein-Izsak, C., Regenerating fish optic nerves and regeneration-like response in injured optic nerve of adult rabbits, Science, **228** (1985) 600-603.
22. Selmaj, K.W. and Ramie, C.S., Tumor necrosis factor mediates myelin and oligodendrocytes damage in vitro, Ann.Neurol., **23** (1988) 339-346.
23. Sommer, I. and Schachner, M., Monoclonal antibodies (O1 to O4) to oligodendrocyte cell surfaces: An immunocytological study in the central nervous system, Dev.Biol., **83** (1981) 311-327.

## Claims

1. An oligodendrocyte cytotoxic factor from lower non-mammalian vertebrates having the following characteristics:
i. it is selectively toxic to oligodendrocytes, but not to other cells, such as type-1 astrocytes and fibroblast cells;
ii. it reduces the number of mature oligodendrocytes in cultures of injured nerves of lower vertebrates and of mammals;
iii. it inhibits differentiation of progenitors of the oligodendrocyte lineage to mature oligodendrocytes;
iv. it is water-soluble;
v. it is heat sensitive, losing its activity on heating at 56°C;
vi. it is protease sensitive losing its activity upon digestion with trypsin; and
vii. it is present in, and is purifiable from, the conditioned media of regenerating injured nerves of lower non-mammalian vertebrates, such as fish, but is not present in the conditioned media of intact nerves of lower vertebrates nor in the conditioned media of injured or intact nerves of mammals.

2. A pharmaceutical composition comprising as an active ingredient the oligodendrocyte cytotoxic factor according to claim 1 and a pharmaceutically acceptable carrier.

3. The pharmaceutical composition according to claim 2 for inducing the regeneration of nerves of mammals.

4. The pharmaceutical composition according to claim 2 for inducing regeneration of injured nerves of the central nervous system of mammals.

5. The pharmaceutical composition according to claim 2 wherein the pharmaceutical composition is such that it can be administered to the site of an injury in mammals.

6. The pharmaceutical composition according to claim 5 wherein the administration is by direct injection to the site of the injury in mammals.

7. The pharmaceutical composition according to claim 5 wherein an implant containing the OCF is inserted at the site of the injury.

8. The pharmaceutical composition according to any of claims 3 to 7 wherein the mammal is a human.

9. A process for the production of a factor according to claim 1 which comprises subjecting conditioned media of regenerating nerves of lower non-mammalian vertebrates to separation procedures, preferably chromatography.

10. A process for the preparation of a pharmaceutical composition according to any one of claims 2 to 8, which comprises combining the oligodentrocyte Cytotoxic Factor according to claim 1 and a pharmaceutically acceptable carrier.

## Patentansprüche

1. Für Oligodendrocyten cytotoxischer Faktor aus niederen Wirbeltieren, die keine Säugetiere sind, mit den folgenden Eigenschaften:
i. er ist selektiv toxisch für Oligodendrocyten, nicht jedoch für andere Zellen, wie z.B. Typ-1-Astrocyten und Fibroblastenzellen;
ii. er verringert die Zahl der reifen Oligodendrocyten in Kulturen von verletzten Nerven von niederen Wirbeltieren und Säugetieren;
iii. er hemmt die Differenzierung von Stammzellen der Oligodendrocyten-Familie zu reifen Oligodendrocyten;
iv. er ist wasserlöslich;
v. er ist hitzeempfindlich, wobei er seine Aktivität bei Erhitzen auf 56°C verliert;
vi. er ist empfindlich gegenüber Proteasen, wobei er seine Aktivität bei Spaltung durch Trypsin verliert; und
vii. er liegt in den konditionierten Medien von sich regenerierenden verletzten Nerven von niederen Wirbeltieren, die keine Säugetiere sind, wie z.B. Fischen, vor und kann daraus gereinigt werden, dagegen liegt er weder in den konditionierten Medien von intakten Nerven von niederen Wirbeltieren noch in den konditionierten Medien von verletzten oder intakten Nerven von Säugetieren vor.

2. Arzneimittel, das als Wirkstoff den für Oligodendrocyten cytotoxischen Faktor nach Anspruch 1 und einen pharmazeutisch verträglichen Träger umfaßt.

3. Arzneimittel nach Anspruch 2 für die Induktion der Regeneration von Nerven von Säugetieren.

4. Arzneimittel nach Anspruch 2 für die Induktion der Regeneration von verletzten Nerven des Zentralnervensystems von Säugetieren.

5. Arzneimittel nach Anspruch 2, wobei das Arzneimittel so beschaffen ist, daß es an der Stelle der Verletzung in Säugetieren verabreicht werden kann.

6. Arzneimittel nach Anspruch 5, wobei die Verabreichung durch eine direkte Injektion an die Stelle der Verletzung in Säugetieren durchgeführt wird.

7. Arzneimittel nach Anspruch 5, wobei ein Implantat, das den OCF enthält, an der Stelle der Verletzung eingepflanzt wird.

8. Arzneimittel nach einem der Ansprüche 3 bis 7, wobei das Säugetier ein Mensch ist.

9. Verfahren zur Herstellung eines Faktors nach Anspruch 1, umfassend die Auftrennung von konditionierten Medien von sich regenerierenden Nerven von niederen Wirbeltieren, die keine Säugetiere sind, mittels Trennverfahren, vorzugsweise Chromatographie.

10. Verfahren zur Herstellung eines Arzneimittels nach einem der Ansprüche 2 bis 8, umfassend die Kombination des für Oligodendrocyten cytotoxischen Faktors nach Anspruch 1 und eines pharmazeutisch verträglichen Trägers.

## Revendications

1. Facteur cytotoxique d'oligodendrocyte de vertébré inférieur non mammifère, présentant les caractéristiques suivantes :
i. il est sélectivement toxique vis-à-vis des oligodendrocytes, mais non toxique vis-à-vis des autres cellules telles que les astrocytes de type 1 et les cellules de fibroblaste ;
ii. il réduit le nombre d'oligodendrocytes matures dans les cultures de nerfs endommagés de vertébré inférieur et de mammifère ;
iii. il inhibe la différentiation des progéniteurs de la lignée oligodendrocytaire en oligodendrocytes matures ;
iv. il est hydrosoluble;
v. il est sensible à la chaleur, perdant son activité à une température de 56°C ;
vi. il est sensible aux protéases, perdant son activité par digestion avec la trypsin ; et
vii. il est présent dans et purifiable de milieux conditionnés de régénération de nerfs endommagés de vertébré inférieur non-mammifère tel que le poisson, mais n'est pas présent dans les milieux conditionnés de nerfs intacts de vertébré inférieur ni dans les milieux conditionnés de nerfs endommagés ou intacts de mammifère.

2. Composition pharmaceutique comprenant comme ingrédient actif le facteur cytotoxique d'oligodendrocyte selon la revendication 1 et un véhicule pharmaceutiquement acceptable.

3. Composition pharmaceutique selon la revendication 2 pour induire la régénération de nerfs de mammifère.

4. Composition pharmaceutique selon la revendication 2 pour induire la régénération de nerfs endommagés du système nerveux central de mammifère.

5. Composition pharmaceutique selon la revendication 2, dans laquelle la composition pharmaceutique est telle qu'elle peut être administrée au niveau (ou site) de la lésion chez le mammifère.

6. Composition pharmaceutique selon la revendication 5, dans laquelle l'administration est effectuée par injection directe au niveau de la lésion chez le mammifère.

7. Composition pharmaceutique selon la revendication 5, dans laquelle un implant contenant le facteur cytotoxique d'oligodendrocyte est introduit au niveau de la lésion.

8. Composition pharmaceutique selon l'une quelconque des revendications 3 à 7, dans laquelle le mammifère est un être humain.

9. Procédé pour la production d'un facteur selon la revendication 1, ledit procédé comprenant la soumission d'un milieu conditionné de régénération de nerfs de vertébré inférieur non-mammifère à des étapes de séparation, de préférence par chromatographie.

10. Procédé pour la préparation d'une composition pharmaceutique selon l'une quelconque des revendications 2 à 8, ledit procédé comprenant la combinaison du facteur cytotoxique d'oligodendrocyte selon la revendication 1 à un véhicule pharmaceutiquement acceptable.
